# EUROPÄISCHE PATENTSCHRIFT

(11) **EP 1 684 071 B1**
(45) Veröffentlichungstag und Bekanntmachung des Hinweises auf die Patenterteilung: **30.03.2011**
(21) Anmeldenummer: 05027277.2
(22) Anmeldetag: 14.12.2005
(51) Int. Cl.: G01N 33/50, C12Q 1/56

(54) **Stabiles, chromogenes Testreagenz und dessen Verwendung in gerinnungsdiagnostischen Tests**
Stable, chromogenic test reagent and its use in tests for diagnosis of coagulation
Réactif de test stable et chromogènes et son utilisation dans des tests diagnostiques de coagulation

(30) Priorität: 21.01.2005 DE 102005003145
(43) Veröffentlichungstag der Anmeldung: 26.07.2006
(73) Patentinhaber: Siemens Healthcare Diagnostics Products GmbH, 35041 Marburg (DE)
(72) Erfinder: Henckel, Thilo, Dr., 35083 Wetter (DE)

(56) Entgegenhaltungen:
- EP-A- 0 420 332
- EP-A- 0 456 152
- EP-A- 0 802 986
- US-A- 4 598 043
- CHROMOGENIX INSTRUMENTATION LABORATORY: "COAMATIC LR Antithrombin" INTERNET ARTICLE - PRODUCT INFORMATION, [Online] April 2003 (2003-04), XP002366214 Gefunden im Internet: URL:http://www.chromogenix.com/Products/pd f_ins/INS_EN_82%202957%2063.pdf>
- CHROMOGENIX INSTRUMENTATION LABORATORY: "S-2772" INTERNET ARTICLE - PRODUCT INFORMATION, [Online] April 2003 (2003-04), XP002366215 Gefunden im Internet: URL:http://www.chromogenix.com/Products/pd f_ins/INS_EN_82%203013%2063.pdf>
- PENTAPHARM: "Pefachrome PAP (Pefa-5920)" INTERNET ARTICLE - PRODUCT INFORMATION, [Online] 15. April 2003 (2003-04-15), XP002366216 Gefunden im Internet: URL:http://www.pentapharm.com/graphics/Pen tapharm/download/diagnostics/Chromogenic_S ubstrates/Pefachrome_PAP-5920.pdf> [gefunden am 2006-02-06]
- TOMASZEK T A ET AL: "CHROMOPHORIC PEPTIDE SUBSTRATES FOR THE SPECTROPHOTOMETRIC ASSAY OF HIV-1 PROTEASE" BIOCHEMICAL AND BIOPHYSICAL RESEARCH COMMUNICATIONS, ACADEMIC PRESS INC. ORLANDO, FL, US, Bd. 168, Nr. 1, 16. April 1990 (1990-04-16), Seiten 274-280, XP001160901 ISSN: 0006-291X

## Beschreibung

Die vorliegende Erfindung betrifft ein chromogenes Testreagenz, das sich besonders zur Verwendung bei gerinnungsdiagnostischen Tests eignet und das sich dadurch auszeichnet, dass es im flüssigen Zustand eine hohe Stabilität bzw. Haltbarkeit aufweist.

Eine Vielzahl diagnostischer Tests beruht auf der Verwendung chromogener Substrate. Für die Bestimmung von Faktoren mit Proteaseaktivität, wie z. B. für die Bestimmung von Gerinnungsfaktoren in Blut- oder Plasmaproben, werden üblicherweise chromogene Peptidsubstrate verwendet, die aus einem spezifischen Oligo- bzw. Polypeptidanteil und einem Chromophor- (Farbstoffträger-) Anteil bestehen. Das zunächst farblose chromogene Peptidsubstrat wird in Abhängigkeit von der Menge bzw. der Aktivität des in der Probe enthaltenen proteolytischen Faktors gespalten, wodurch das Chromophor freigesetzt wird, welches dann photometrisch gemessen werden kann. In den Patentdokumenten EP 34 122 A1 und US 4,508,644 sind eine Vielzahl von chromogenen Peptidsubstraten und deren Verwendung in diagnostischen Tests beschrieben, z. B. zur Bestimmung der Gerinnungsfaktoren Faktor IIa (Thrombin) und Xa. In dem Dokument EP 78 764 A1 ist ein chromogenes Verfahren zur Bestimmung des Gerinnungsfaktors Xlla beschrieben. In dem Dokument EP 420 332 A2 ist ein chromogenes Verfahren zur Bestimmung des Gerinnungsfaktors IIa (Thrombin) beschrieben.

In dem Dokument EP 0 408 075 A2 wird ein Verfahren zur Bestimmung von Antithrombin III in Körperflüssigkeiten beschrieben. Das zugehörige Testreagenz enthält ein chromogenes Peptidsubstrat und ein Tetrapeptid (Gly-Pro-Arg-Pro) als Inhibitor der Fibrinpolymerisation.

Besonders häufig verwendete Chromophore sind z. B. para-Nitroanilin (pNA) oder 5-Amino-2-Nitro-Benzoesäure (ANBA), deren gelbe Farbe bei einer Wellenlänge von λ= 405 nm messbar ist.

In der Gerinnungsdiagnostik wird die Bestimmung der Aktivität von enzymatisch aktiven Faktoren in Blut- oder Plasmaproben üblicherweise unter möglichst physiologischen Bedingungen durchgeführt. Besonders wichtig ist es, dass ein physiologischer pH-Wert von etwa 7,4 im Testansatz gewährleistet ist. Um pH-Wert Änderungen im Testansatz zu verhindern, sind die Testsubstanzen, die mit der Blut- bzw. Plasmaprobe vermischt werden müssen, üblicherweise in Puffern mit neutralem bis leicht basischem pH-Wert gelöst.

Der Nachteil eines solchen Testdesigns besteht allerdings darin, dass einige Testkomponenten in neutralem bis basischem Milieu eine verringerte Stabilität aufweisen. So weist z. B. pNA bei einem pH-Wert von > 6,0 einen beschleunigten Zerfall auf. Die resultierende Gelbfärbung der pNA-Substratlösung macht eine weitere Verwendung der Lösung bzw. eine zuverlässige Testauswertung unmöglich. Aus diesem Grund werden pNA-Substrate für die Langzeitlagerung üblicherweise lyophilisiert und erst bei Bedarf in einer neutralen Flüssigkeit gelöst. Um den Zerfall von pNA-Substraten in Lösungen zu unterbinden, ist es bekannt, den pH-Wert der Substratlösung sauer einzustellen.

Für diverse chromogene Gerinnungsteste ist es notwendig, die Blutgerinnungskaskade unter Bildung von Thrombin zu aktivieren oder Thrombin direkt zum Testansatz zuzusetzen, jedoch gleichzeitig die Bildung eines Fibringerinnsels zu unterbinden, um Trübungen, die die photometrische Messung der Probe beeinträchtigen würden, vorzubeugen. Dies kann zum Beispiel durch die Verwendung von Inhibitoren der Fibrinpolymerisation im Testansatz bewerkstelligt werden. Bei diesen sog. Clot-Inhibitoren handelt es sich häufig um Oligopeptide, die die Aneinanderlagerung (Polymerisation) der Fibrinmonomere, die unter der Einwirkung von Thrombin entstehen, inhibieren und damit die Gerinnselbildung verhindern (siehe z. B. EP 0 456 152 B1).

Zu berücksichtigende Kriterien bei der Entwicklung von Reagenzien, die die für ein Testverfahren erforderlichen Komponenten bereitstellen, sind u. a. eine einfache Handhabung und eine möglichst lange Haltbarkeit. Besonders bevorzugt sind langzeitstabile flüssige Reagenzformulierungen, da es bei der Rekonstitution lyophilisierter Komponenten durch den Anwender zu Fehlern kommen kann, die sich negativ auf die Qualität des gesamten Testsverfahrens auswirken können.

Der vorliegenden Erfindung lag die Aufgabe zugrunde ein insbesondere im flüssigen Zustand langzeitstabiles Reagenz bereitzustellen, das sowohl ein chromogenes Peptidsubstrat wie auch einen Clot-Inhibitor enthält und sich damit besonders zur Verwendung in chromogenen Gerinnungstesten eignet, die photometrisch ausgewertet werden.

Die Lösung der Aufgabe besteht in der Bereitstellung der in den Ansprüchen beschriebenen erfindungsgemäßen Verfahren und Gegenständen.

Die vorliegende Erfindung betrifft im wesentlichen eine Flüssigzubereitung, die ein chromogenes Peptidsubstrat enthält und einen Inhibitor der Fibrinpolymerisation, welcher aus einem Oligopeptid besteht, das die Polymerisation von Fibrinmonomeren, welche unter der Einwirkung von Thrombin entstehen, inhibiert und weiterhin dadurch gekennzeichnet ist, dass der pH-Wert der Zubereitung zwischen 3,0 und 6,0, bevorzugt zwischen 4,0 und 5,0 liegt. In einer besonders bevorzugten Ausführungsform beträgt der pH-Wert des Reagenzes 4,6 ± 0,1.

Zur Einstellung des pH-Wertes werden der flüssigen, wässrigen Zubereitung Protonenspender zugesetzt, d. h. Säuren bzw. deren Salze. Es können anorganische oder organische Säuren bzw. Protonenspender verwendet werden. Ein geeignetes Puffersystem kann notwendig sein, um einen stabilen pH-Wert zu gewährleisten und um z. B. eine Alkalisierung durch Kohlendioxid zu verhindern.

Eine besonders bevorzugte Ausführungsform einer erfindungsgemäßen Zubereitung enthält Acetat-Ionen (CH₃COO⁻). Ein weiterer Gegenstand der vorliegenden Erfindung ist daher ein Verfahren zur Herstellung eines Testreagenzes, wobei ein chromogenes Peptidsubstrat und ein Inhibitor der Fibrinpolymerisation, welcher aus einem Oligopeptid besteht, das die Polymerisation von Fibrinmonomeren, welche unter der Einwirkung von Thrombin entstehen, inhibiert, kombiniert werden und der pH-Wert des Reagenzes mit Essigsäure auf einen Wert zwischen 3,0 und 6,0, bevorzugt auf einen Wert zwischen 4,0 und 5,0, besonders bevorzugt auf einen Wert von 4,6 ± 0,1 eingestellt wird.

Überraschenderweise verfügt eine erfindungsgemäße Zubereitung im flüssigen Zustand über eine höhere Stabilität und somit längere Lagerungsfähigkeit als eine entsprechende Zubereitung mit neutralem pH-Wert in flüssigem oder lyophilisiertem Zustand.

Es konnte beobachtet werden, dass eine erfindungsgemäße Zubereitung im flüssigen Zustand über einen Zeitraum von 60 Wochen bei einer Lagerungstemperatur von 4 °C eine Messwertabweichung vom Initialwert von nicht mehr als 10 % aufweist. Als Initialwert ist der Messwert oder das Testergebnis zu verstehen, der/das unter Verwendung der Zubereitung als Testreagenz am Herstellungstag der Zubereitung in einem geeigneten Testverfahren ermittelt wurde. Als Verfahren zur Bestimmung der Stabilität eines Reagenzes, das beispielsweise ein chromogenes Peptidsubstrat enthält, welches von Thrombin gespalten wird, eignet sich z. B. ein ETP-Test (siehe EP 0 420 332 B1 bzw. Beispiel 2 unten).

Chromogene Peptidsubstrate im Sinne der vorliegenden Erfindung bestehen aus einem spezifischen Oligo- bzw. Polypeptidanteil, an welchen eine chromogene Gruppe, d. h. eine abspaltbare, farbige oder fluoreszierende Gruppe gekoppelt ist, die nach Abspaltung vom Peptidsubstrat andere optische Eigenschaften aufweist als das ungespaltene chromogene Peptidsubstrat und absorptions- bzw. fluoreszenzspektrophotometrisch gemessen werden kann. Beispiele für chromogene Gruppen, die an ein Peptidsubstrat gekoppelt sein können, sind para-Nitroanilin (pNA), 5-Amino-2-Nitro-Benzoesäure (ANBA), 7-Amino-4-Methoxy Coumarin (AMC), Chinonylamid (ChA), 5-Amino-Isophtalsäure-Dimethylester (DPA) und deren Derivate.

Das chromogene Peptidsubstrat ist im Hinblick auf den Oligo- bzw. Polypeptidanteil aus der dem Fachmann bekannten Vielzahl an Substraten, die von dem zu bestimmenden Gerinnungsfaktor, wie z. B. von Faktor X/Xa oder von Thrombin gespalten werden, wählbar. In einer bevorzugten Ausführungsform enthält das Reagenz ein chromogenes Peptidsubstrat, das von Thrombin erkannt und umgesetzt wird, wie z. B. ein Peptidsubstrat der Sequenz Ala-Gly-Arg-R₁ oder der Sequenz Gly-Gly-Arg-R₁ , wobei R₁ für eine chromogene Gruppe steht. Besonders bevorzugte Peptidsubstrate, die von Thrombin erkannt und umgesetzt werden, sind z. B. das pNA-gekoppelte Peptidsubstrat Ala-Gly-Arg-pNA (Pefachrome®TG, Pentapharm Ltd., Basel, Schweiz) oder das AMC-gekoppelte Peptidsubstrat Gly-Gly-Arg-AMC (Bachem). Weitere geeignete Peptidsubstrate, die von Thrombin gespalten werden, sind solche der allgemeinen Formel Msc-Val-Xaa-R₁, worin Msc für Methylsulfonylethyloxycarbonyl, Val für die Aminosäure Valin, und Xaa für einen Aminosäurerest, der eine terminale Guanidino-Gruppe oder Ureido-Gruppe umfasst, getrennt durch mindestens zwei Kohlenstoffatome vom Peptidgerüst, steht und worin R₁ für eine chromogene Gruppe steht, wobei das Peptid Msc-Val-Arg-R₁ bzw. Msc-Val-Arg-pNA besonders bevorzugt ist (EP 0 802 986 B1). Weitere Beispiele für chromogene Peptidsubstrate mit Spezifität für verschiedene Proteasen finden sich z. B. in dem US Patent 4,508,644.

In einer bevorzugten Ausführungsform kann die Zubereitung zusätzlich einen oder mehrere Stabilisatoren enthalten, wie z. B. bovines Serumalbumin, chaotrope Salze, Chaperone und chaperone-ähnliche (chaperone-like) Substanzen, Dextran und andere Zucker.

Als Inhibitoren der Fibrinpolymerisation eignen sich Oligopeptide, welche die Polymerisation von Fibrinmonomeren, welche unter der Einwirkung von Thrombin entstehen, inhibieren, besonders bevorzugt solche der allgemeinen Peptidsequenz GPRP-X-NH₂, wobei G für die Aminosäure Glycin, P für die Aminosäure L-Prolin, R für die Aminosäure L-Arginin und X für Alanin oder Glycin steht (siehe EP 0 456 152 B1).

Die Erfindung betrifft weiterhin die Verwendung der erfindungsgemäßen Zubereitung als Testreagenz in einem Testverfahren zur Bestimmung eines Gerinnungsparameters in einer Vollblut- oder Plasmaprobe. Als Probenmaterial eignet sich Vollblut oder Plasma, humaner oder tierischer Herkunft. Besonders gut eignet sich plättchenarmes oder plättchenreiches Plasma, welches mit EDTA und/oder Citrat versetzt sein kann.

Bevorzugt ist die Verwendung einer erfindungsgemäßen Zubereitung als Testreagenz in einem Testverfahren zur Bestimmung der Thrombingenerierung in Blut oder Plasma. Derartige Testverfahren sind z. B. in den Patentdokumenten EP 1 367 135 A1 und EP 0 420 332 B1 beschrieben. Besonders bevorzugt ist die Verwendung des erfindungsgemäßen Reagenzes in einem Testverfahren zur Bestimmung des endogenen Thrombinpotenzials (ETP) und weiterer Gerinnungsparameter, welche aus der Thrombinbildungskurve berechnet werden können oder aus der gemessenen Umsatzkinetik eines chromogenen Thrombinsubstrats abgeleitet werden können [siehe EP 0 420 332 B1, EP 0 802 986 B1 und Hemker et al. (1993) Thromb. Haemostasis 70: 617-624].

Weiterhin bevorzugt ist die Verwendung einer erfindungsgemäßen Zubereitung als Testreagenz in chromogenen Testen zur Bestimmung der Gerinnungszeit, wie z. B. in einem chromogenen Prothrombinzeit (PT)-Gerinnungstest wie in dem Patentdokument EP 0 014 039 A1 beschrieben.

Ein weiterer Gegenstand der Erfindung ist ein Testkit zur Durchführung eines chromogenen Testverfahrens, wobei das Testkit mindestens eine Flüssigzubereitung enthält, die ein chromogenes Peptidsubstrat enthält und einen Inhibitor der Fibrinpolymerisation, welcher aus einem Oligopeptid besteht, das die Polymerisation von Fibrinmonomeren, welche unter der Einwirkung von Thrombin entstehen, inhibiert, und einen pH-Wert zwischen 3,0 und 6,0 aufweist. Ein bevorzugtes Testkit zur Verwendung in einem gerinnungsdiagnostischen Testverfahren enthält zusätzlich ein oder mehrere Gerinnungsaktivatoren. Um beispielsweise die Thrombinbildung zu induzieren, können z. B. Lösungen oder Lyophilisate in dem Testkit enthalten sein, die Ca²⁺-Ionen, Thromboplastin oder Kontaktaktivatoren, wie z. B. Kaolin, Phospholipide, Schlangengift, oder Thrombomodulin und aktiviertes Protein C enthalten.

### Figuren

Die Figuren 1 bis 3 sind graphische Darstellungen von Testergebnissen (absolute Messwerte), die mit einer erfindungsgemäßen sauren Flüssigzubereitung (pH 4,6) bzw. mit neutralen Zubereitungen (pH 7,4) gemessen wurden.
Figur 1: Lagerung der Zubereitungen bei + 4 °C.
Figur 2: Lagerung der Zubereitungen bei Raumtemperatur.
Figur 3: Lagerung der Zubereitungen bei + 37 °C.

Die folgenden Beispiele dienen der Veranschaulichung der Erfindung und sind nicht als Einschränkung zu verstehen.

### Beispiele

### Beispiel 1: Herstellung eines erfindungsgemäßen Reagenzes

Zur Herstellung eines erfindungsgemäßen Testreagenzes, das sich für die Verwendung in einem Verfahren zur Bestimmung der Thrombinaktivität eignet, wurde gefriergetrocknetes H-β-Ala-Gly-Arg-pNA·2AcOH als chromogenes Thrombinsubstrat (Pefachrome®, Pentapharm Ltd., Basel, Schweiz), gefriergetrocknetes H-Gly-Pro-Arg-Pro-Ala-NH₂ (als Trifluoressigsäuresalz) als Clot-Inhibitor, NaCl, bovines Serumalbumin und Dextran in demineralisiertem Wasser gelöst, und der pH-Wert der Lösung wurde mit Essigsäure auf einen Wert von 4,6 ± 0,1 eingestellt. Das Reagenz wurde schließlich in ein verschließbares Reagenzbehältnis abgefüllt und im flüssigen Zustand aufbewahrt.

Zu Vergleichszwecken wurden Reagenzien mit neutralem pH-Wert hergestellt. Dazu wurde gefriergetrocknetes Msc-Val-Arg-pNA·2HCl als chromogenes Thrombinsubstrat (Neosystem Groupe SNPE, Strasbourg, Frankreich) und gefriergetrocknetes H-Gly-Pro-Arg-Pro-Ala-NH₂ (als Trifluoressigsäuresalz) als Clot-Inhibitor, NaCl, Tris/HCl, bovines Serumalbumin und Dextran in demineralisiertem Wasser gelöst und der pH-Wert auf 7,4 eingestellt. Ein Teil dieses neutralen Reagenzes wurde in ein verschließbares Reagenzbehältnis abgefüllt und im flüssigen Zustand aufbewahrt, während der andere Teil in ein Lyophilisationsgefäß transferiert wurde, gefriergetrocknet wurde und als Lyophilisat aufbewahrt wurde.

### Beispiel 2: Verwendung eines erfindungsgemäßen Reagenzes in einem Testverfahren zur Bestimmung des endogenen Thrombinpotenzials (ETP) nach Langzeitlagerung unter verschiedenen Bedingungen

Zur Überprüfung der Stabilität des erfindungsgemäßen sauren Flüssigreagenzes wurden Aliquots des Reagenzes bis zu 96 Wochen bei 4 °C, bei Raumtemperatur (RT, 15-25 °C) bzw. bei 37 °C eingelagert und nach unterschiedlichen Lagerungsperioden in einem ETP-Test eingesetzt. Mit den beiden neutralen Kontrollreagenzformulierungen wurde analog verfahren.

Als Probenmaterial wurde in allen Versuchen eine Charge tiefgefrorenes, plättchenarmes Humanplasma (Normalplasmapool) verwendet, das zum jeweiligen Testzeitpunkt aliquotweise aufgetaut wurde.

Mit dem erfindungsgemäßen sauren Reagenz wurde ein ETP-Test folgendermaßen durchgeführt:

135 µl Plasma wurden zunächst mit 40 µl Puffer (50 mM Tris-HCl, pH 7,4) vermischt. Anschließend wurden 40 µl des erfindungsgemäßen Reagenzes zugegeben. Nach einer 7minütigen Inkubation wurden dem Ansatz 15 µl CaCl₂ (250 mM) und 30 µl Innovin^{®} (Reagenz bestehend aus rekombinantem, humanem Gewebefaktor und einer Mischung von synthetischen Phospholipiden; Dade Behring Marburg GmbH, Marburg, Deutschland) zur Aktivierung der Thrombinbildung zugegeben. Über einen Zeitraum von 20 Minuten wurde die Absorption des Testansatzes bei einer Wellenlänge von λ= 405 nm kontinuierlich gemessen. Anhand der so bestimmten Umsatzkinetik des chromogenen Thrombinsubstrats wurde mit Hilfe eines Auswerteverfahrens, beschrieben in der Patentanmeldung DE 10 2004 059 055.9 der ETP-Wert der Probe bestimmt.

Das Mischen der Reagenzien mit der Probe, die Absorptionsmessung und die automatische Bestimmung des ETP-Werts erfolgten vollautomatisch auf einem BCS^{®} Gerinnungsanalyzer (Dade Behring Marburg GmbH, Marburg, Deutschland).

Mit den neutralen Kontrollreagenzien wurde ein ETP-Test folgendermaßen durchgeführt (siehe auch EP 0 420 332 B1):

Das lyophilisierte Reagenz wurde zum jeweiligen Testzeitpunkt mit demineralisiertem Wasser frisch gelöst. 200 µl Plasma wurden mit 80 µl des flüssigen neutralen bzw. mit 80 µl des frisch rekonstituierten lyophilisierten Reagenzes vermischt. Nach einer 7minütigen Inkubation wurden dem Ansatz 30 µl CaCl₂ (250 mM) und 60 µl Innovin^{®} (Dade Behring Marburg GmbH, Marburg, Deutschland) zur Aktivierung der Thrombinbildung zugegeben. Die Messung der Absorption und die Bestimmung des ETP-Werts erfolgte wie oben beschrieben.

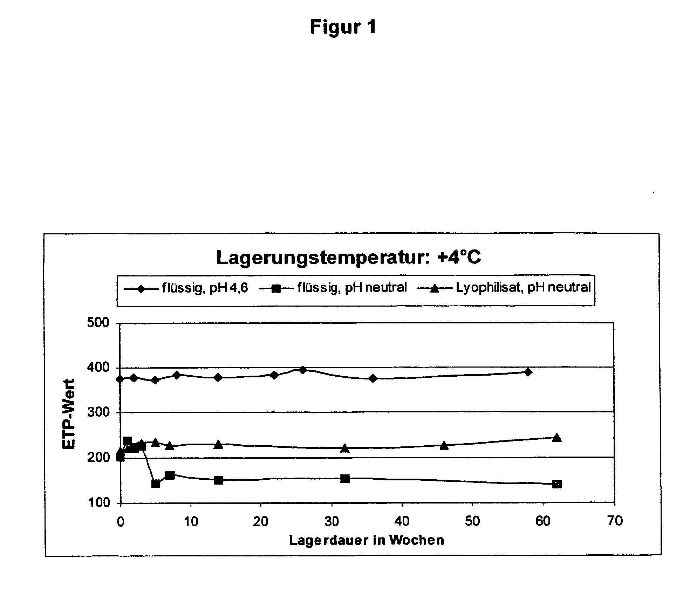

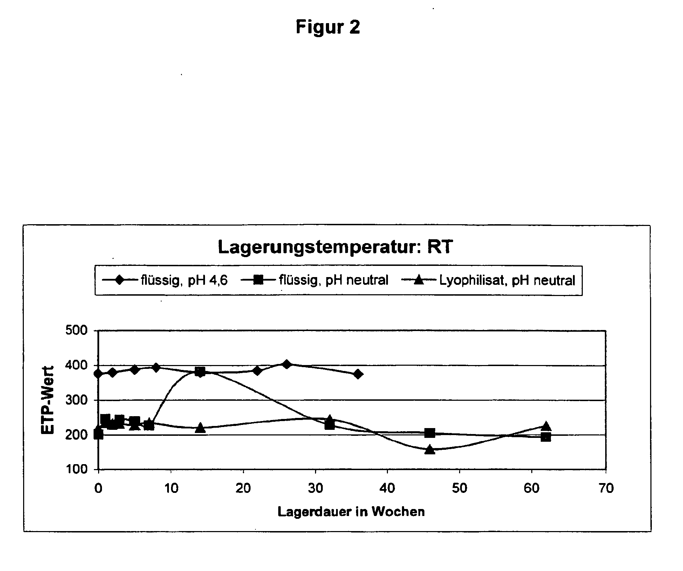

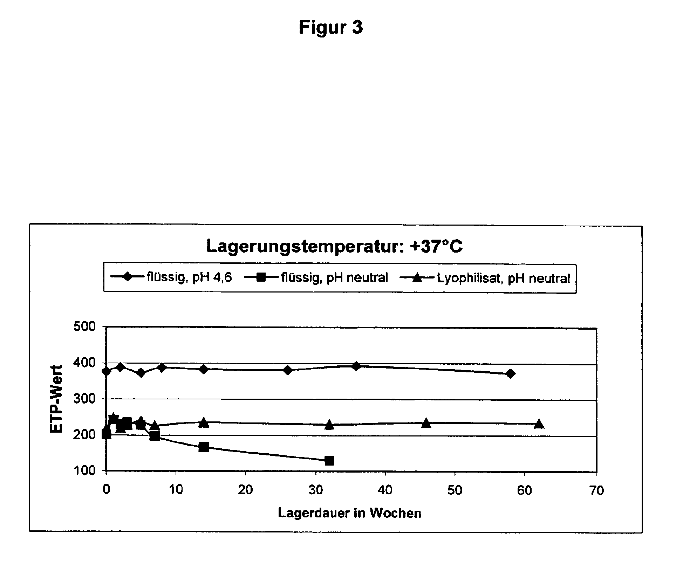

Die Testergebnisse sind in Abhängigkeit von der Lagerungsdauer der Reagenzien bei den verschiedenen Lagerungstemperaturen 4 °C (Fig. 1), Raumtemperatur (15-25 °C) (Fig. 2) bzw. 37 °C (Fig. 3) in den Figuren 1 bis 3 graphisch dargestellt.

Wie aus den Abbildungen hervorgeht, zeichnet sich die erfindungsgemäße Reagenzformulierung durch eine hohe Stabilität über einen Zeitraum von bis zu 60 Wochen aus. Bei allen Lagerungstemperaturen und zu jedem Zeitpunkt liegt die maximale Abweichung vom Initialwert, der zum Zeitpunkt t=0 (Herstellungstag) gemessen wurde, deutlich unter 10 %, vorwiegend sogar unter 5 %. Die lyophilisierte neutrale Reagenzformulierung liefert nach 60wöchiger Lagerung bei 4 °C eine Messwertabweichung vom Initialwert von über 10 % und bei erhöhter Lagerungstemperatur (37 °C) weicht der Messwert bereits nach 4 Wochen um bis zu 15 % ab. Die flüssige neutrale Reagenzformulierung zeigt bei allen Lagerungstemperaturen sehr große Schwankungen von bis zu mehr als 20 % und ist demnach nicht stabil. Die Haltbarkeit des flüssigen neutralen Reagenzes ist auf einen, maximal zwei Tage beschränkt.

In Tabelle 1 sind die prozentualen Abweichungen vom jeweiligen Initialmesswert zum Zeitpunkt t₀ der verschiedenen Reagenzformulierungen nach unterschiedlichen Lagerungsbedingungen aufgeführt. Die verschiedenen Reagenzformulierungen wurden über einen Zeitraum von bis zu 96 Wochen bei 2 bis 8 °C, bei Raumtemperatur bzw. bei 37 °C gelagert. Bei einer Lagerungstemperatur von 2 bis 8°C weichen die Messwerte, die mit einer erfindungsgemäßen Zubereitung über den gesamtem Zeitraum ermittelt wurden (flüssig, pH 4,6) nicht mehr als 5 % ab. Bei allen untersuchten Lagerungstemperaturen weist die saure flüssige Zubereitung die geringsten Abweichungen und damit die höchste Stabilität auf. Bei der neutralen Flüssigzubereitung (flüssig, pH 7,4) wurde mit andauernder Lagerung bei Raumtemperatur bzw. bei 37 °C eine zunehmende Gelbfärbung des Reagenzes sichtbar.

**Tabelle 1**

| **Abweichung vom Initialwert in Prozent [%]** | | | | | | | | | | | | | | |
|---|---|---|---|---|---|---|---|---|---|---|---|---|---|---|
| **Lagerungstemperatur [°C]** | **Zeit t [Wochen]** | **1** | **2** | **5** | **7** | **8** | **14** | **26** | **32** | **36** | **46** | **58** | **82** | **96** |
| 2-8 °C | **flüssig, pH 4,6** | | **0** | **1** | | -2 | **0** | **-5** | | **0** | | **-4** | **3** | **0** |
| | flüssig, pH neutral | -17 | -12 | 29 | 20 | | 25 | | 24 | | | | | |
| | Lyophilisat, pH neutral | -2 | -3 | -8 | -5 | | -5 | | -2 | | -5 | | | |
| Raumtemperatur | **flüssig, pH 4,6** | | **-1** | **-3** | | **-5** | **-1** | **-7** | | **0** | | | | |
| | flüssig, pH neutral | -22 | -14 | -19 | -13 | | -90 | | -13 | | -2 | | | |
| | Lyophilisat, pH neutral | -9 | -7 | -5 | -8 | | -2 | | -12 | | 27 | | | |
| 37 °C | **flüssig, pH 4,6** | | **-3** | **1** | | **-3** | **-2** | **-1** | | **-4** | | **1** | | |
| | flüssig, pH neutral | -20 | -13 | -13 | 2 | | 18 | | 36 | | | | | |
| | Lyophilisat, pH neutral | -14 | -1 | -10 | -4 | | -8 | | -6 | | -8 | | | |

## Patentansprüche

1. Flüssigzubereitung, enthaltend
a) ein chromogenes Peptidsubstrat und
b) einen Inhibitor der Fibrinpolymerisation bestehend aus einem Oligopeptid, das die Polymerisation von Fibrinmonomeren, welche unter der Einwirkung von Thrombin entstehen, inhibiert,
**dadurch gekennzeichnet, dass** der pH-Wert der Zubereitung zwischen 3,0 und 6,0 liegt.

2. Zubereitung nach Anspruch 1 **dadurch gekennzeichnet, dass** Acetat-Ionen enthalten sind.

3. Zubereitung nach Anspruch 1 oder 2 **dadurch gekennzeichnet, dass** als chromogenes Peptidsubstrat ein para-Nitroanilin gekoppeltes Peptidsubstrat enthalten ist.

4. Zubereitung nach Anspruch 1 oder 2 **dadurch gekennzeichnet, dass** als chromogenes Peptidsubstrat ein 5-Amino-2-Nitro-Benzoesäure gekoppeltes Peptidsubstrat enthalten ist.

5. Zubereitung nach einem der Ansprüche 1 bis 4 **dadurch gekennzeichnet, dass** ein chromogenes Peptidsubstrat enthalten ist, das von Thrombin gespalten wird.

6. Zubereitung nach Anspruch 5 **dadurch gekennzeichnet, dass** ein chromogenes Peptidsubstrat der Sequenz Ala-Gly-Arg-R₁ enthalten ist, wobei R₁ für eine chromogene Gruppe steht.

7. Zubereitung nach Anspruch 5 **dadurch gekennzeichnet, dass** ein chromogenes Peptidsubstrat der Sequenz Msc-Val-Arg-R₁ enthalten ist, wobei Msc für Methylsulfonylethyloxycarbonyl und R₁ für eine chromogene Gruppe steht.

8. Zubereitung nach einem der Ansprüche 1 bis 7 **dadurch gekennzeichnet, dass** ein Inhibitor der Fibrinpolymerisation der allgemeinen Peptidsequenz GPRP-X-NH₂ enthalten ist, wobei G für die Aminosäure Glycin, P für die Aminosäure L-Prolin, R für die Aminosäure L-Arginin und X für Alanin oder Glycin steht.

9. Zubereitung nach einem der Ansprüche 1 bis 8 **dadurch gekennzeichnet, dass** zusätzlich ein oder mehrere Stabilisatoren enthalten sind.

10. Verfahren zur Herstellung einer Zubereitung gemäß einem der Ansprüche 1 bis 9 **dadurch gekennzeichnet, dass** der pH-Wert der Zubereitung durch Zugabe eines Protonenspenders auf einen Wert zwischen 3,0 und 6,0 eingestellt wird.

11. Verfahren gemäß Anspruch 10 **dadurch gekennzeichnet, dass** der pH-Wert der Zubereitung durch Zugabe von Essigsäure auf einen Wert zwischen 3,0 und 6,0 eingestellt wird.

12. Verwendung einer Zubereitung gemäß einem der Ansprüche 1 bis 9 als Testreagenz in einem Testverfahren zur Bestimmung eines Gerinnungsparameters in einer Vollblut- oder Plasmaprobe.

13. Verwendung einer Zubereitung gemäß einem der Ansprüche 1 bis 9 als Testreagenz in einem Testverfahren zur Bestimmung der Thrombingenerierung in einer Vollblut- oder Plasmaprobe.

14. Verwendung einer Zubereitung gemäß einem der Ansprüche 1 bis 9 als Testreagenz in einem Testverfahren zur Bestimmung des endogenen Thrombinpotenzials einer Vollblut- oder Plasmaprobe.

15. Testkit zur Durchführung eines chromogenen Testverfahrens **dadurch gekennzeichnet, dass** es eine Zubereitung gemäß Anspruch 1 enthält.

## Claims

1. A liquid preparation comprising
a) a chromogenic peptide substrate and
b) an inhibitor of fibrin polymerization consisting of an oligopeptide which inhibits the polymerization of fibrin monomers formed under the influence of thrombin,
wherein the pH of the preparation is between 3.0 and 6.0.

2. The preparation as claimed in claim 1, wherein acetate ions are present.

3. The preparation as claimed in claim 1 or 2, wherein the chromogenic peptide substrate present is a para-nitroaniline-coupled peptide substrate.

4. The preparation as claimed in claim 1 or 2, wherein the chromogenic peptide substrate present is a 5-amino-2-nitrobenzoic acid-coupled peptide substrate.

5. The preparation as claimed in one of claims 1 to 4, wherein a chromogenic peptide substrate which is cleaved by thrombin is present.

6. The preparation as claimed in claim 5, wherein a chromogenic peptide substrate of the sequence Ala-Gly-Arg-R₁, where R₁ is a chromogenic group, is present.

7. The preparation as claimed in claim 5, wherein a chromogenic peptide substrate of the sequence Msc-Val-Arg-R₁, where Msc is methylsulfonylethyloxycarbonyl and R₁ is a chromogenic group, is present.

8. The preparation as claimed in one of claims 1 to 7, wherein an inhibitor of fibrin polymerization of the general peptide sequence GPRP-X-NH₂, where G is the amino acid glycine, P is the amino acid L-proline, R is the amino acid L-arginine and X is alanine or glycine, is present.

9. The preparation as claimed in one of claims 1 to 8, wherein one or more stabilizers are additionally present.

10. A method for preparing a preparation as claimed in one of claims 1 to 9, wherein the pH of the preparation is adjusted to a value of between 3.0 and 6.0 by adding a proton donor.

11. The method as claimed in claim 10, wherein the pH of the preparation is adjusted to a value of between 3.0 and 6.0 by adding acetic acid.

12. The use of a preparation as claimed in one of claims 1 to 9 as a test reagent in a test method for determining a coagulation parameter in a whole blood or plasma sample.

13. The use of a preparation as claimed in one of claims 1 to 9 as a test reagent in a test method for determining thrombin generation in a whole blood or plasma sample.

14. The use of a preparation as claimed in one of claims 1 to 9 as a test reagent in a test method for determining the endogenous thrombin potential of a whole blood or plasma sample.

15. A test kit for carrying out a chromogenic test method, comprising a preparation as claimed in claim 1.

## Revendications

1. Préparation liquide, contenant
a) un substrat de peptide chromogène et
b) un inhibiteur de la polymérisation de la fibrine constitué d'un oligopeptide, qui inhibe la polymérisation de monomères de fibrine, qui se forment sous l'effet de la trombine,
**caractérisée en ce que** le pH de la préparation est compris entre 3,0 et 6,0.

2. Préparation suivant la revendication 1, **caractérisée en ce qu'**elle contient des ions acétates.

3. Préparation suivant la revendication 1 ou 2, **caractérisée en ce qu'**elle contient comme substrat de peptide chromogène un substrat de peptide couplé à de la para-nitroaniline.

4. Préparation suivant la revendication 1 ou 2, **caractérisée en ce qu'**elle contient comme substrat de peptide chromogène un substrat de peptide couplé à de l'acide 5-amino-2-nitro-benzoïque.

5. Préparation suivant l'une des revendications 1 à 4, **caractérisée en ce qu'**elle contient un substrat de peptide chromogène, qui est décomposé par de la trombine.

6. Préparation suivant la revendication 5, **caractérisée en ce qu'**elle contient un substrat de peptide chromogène de la séquence Ala-Gly-Arg-R₁, R₁ étant un groupe chromogène.

7. Préparation suivant la revendication 5, **caractérisée en ce qu'**elle contient un substrat de peptide chromogène de la séquence Msc-Val-Arg-R₁, Msc représentant méthylsulfonyl-éthyloxycarbonyle et R₁ un groupe chromogène.

8. Préparation suivant l'une des revendications 1 à 7, **caractérisée en ce qu'**elle contient un inhibiteur de la polymérisation de la fibrine de la séquence de peptide générale GPRP-X-NH₂, G représentant l'acide aminé glycine, P l'acide aminé L-proline, R l'acide aminé L-arginine et X l'alanine ou la glycine.

9. Préparation suivant l'une des revendications 1 à 8, **caractérisée en ce qu'**elle contient en plus un stabilisant ou plusieurs stabilisants.

10. Procédé de production d'une préparation suivant l'une des revendications 1 à 9, **caractérisé en ce qu'**on règle le pH de la préparation à une valeur comprise entre 3,0 et 6,0 par addition d'un donneur de proton.

11. Procédé suivant la revendication 10, **caractérisé en ce qu'**on règle le pH de la préparation à une valeur comprise entre 3,0 et 6,0 par addition d'acide acétique.

12. Utilisation d'une préparation suivant l'une des revendications 1 à 9 comme réactif de test dans un procédé de test pour la détermination d'un paramètre de coagulation d'un échantillon de sang complet ou d'un échantillon de plasma.

13. Utilisation d'une préparation suivant l'une des revendications 1 à 9 comme réactif de test dans un procédé de test pour la détermination de la production de trombine dans un échantillon de sang complet ou dans un échantillon de plasma.

14. Utilisation d'une préparation suivant l'une des revendications 1 à 9 comme réactif de test dans un procédé de test pour la détermination du potentiel de trombine endogène d'un échantillon de sang complet ou d'un échantillon de plasma.

15. Trousse de test pour effectuer un procédé de test chromogène, **caractérisée en ce qu'**elle contient une préparation suivant la revendication 1.
